# EUROPEAN PATENT APPLICATION

(11) **EP 2 380 868 A1**
(43) Date of publication of application: **26.10.2011**
(21) Application number: 09835052.3
(22) Date of filing: 25.12.2009
(51) Int. Cl.: C07C 68/06, C07C 68/08, C07C 69/96, C07B 61/00

(54) **PRODUCING PROCESS DIALKYL CARBONATE**

(30) Priority: 26.12.2008 JP 2008333601; 22.12.2009 JP 2009290722
(71) Applicant: Mitsubishi Chemical Corporation, Tokyo 108-0014 (JP)
(72) Inventor: SHIMIZU Satoshi, Kamisu-shi Ibaraki 314-0102 (JP)
(74) Representative: Vossius & Partner
(86) International application number: PCT/JP2009/071654
(87) International publication number: WO 2010/074256

(57) **Abstract**

An object of the present invention is to provide an industrially advantageous process for simultaneously producing a symmetric dialkyl carbonate and an asymmetric dialkyl carbonate by performing a transesterification reaction of an alkylene carbonate with two or more kinds of alcohols; and a process for efficiently producing diethyl carbonate in high purity by performing transesterification of ethylene carbonate or propylene carbonate with ethanol, The present invention relates to a process for simultaneously producing a symmetric dialkyl carbonate and an asymmetric dialkyl carbonate, comprising performing a transesterification reaction of an alkylene carbonate with two or more kinds of alcohols in the same reactor, and a process for producing diethyl carbonate, comprising performing a transesterification reaction of ethylene carbonate or propylene carbonate with ethanol, wherein the process comprises a step of subjecting the reaction product obtained in the transesterification reaction to extractive distillation using ethylene glycol or propylene glycol as the extraction solvent to separate by distillation a fraction containing an ether compound.

## Description

### TECHNICAL FIELD

The present invention relates to a process for producing a symmetric dialkyl carbonate and an asymmetric dialkyl carbonate by a transesterification reaction of an alkylene carbonate with two or more alcohols.
The present invention also relates to a process for producing diethyl carbonate by a transesterification reaction of ethylene carbonate or propylene carbonate with ethanol.

### BACKGROUND ART

In producing any dialkyl carbonate of diethyl carbonate, ethyl methyl carbonate and dimethyl carbonate by a transesterification reaction of ethylene carbonate or propylene carbonate with ethanol or a mixture of ethanol and methanol, methyl glycol ethers or ethyl glycol ethers are by-produced.

That is, the reaction proceeds according to the following reaction formulae (1) to (4) and at that time, glycol ethers are by-produced by side reactions of the following reaction formulae (5) to (8).

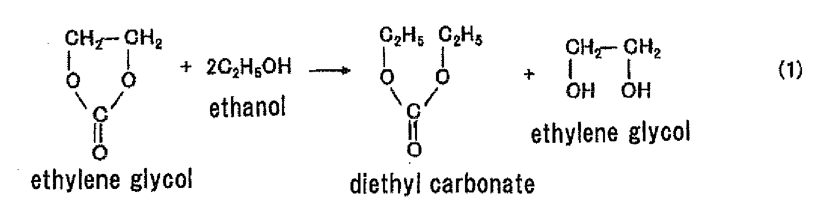

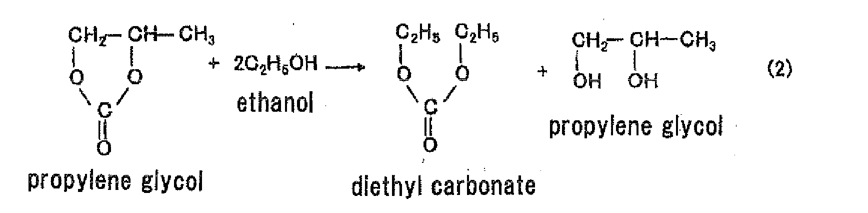

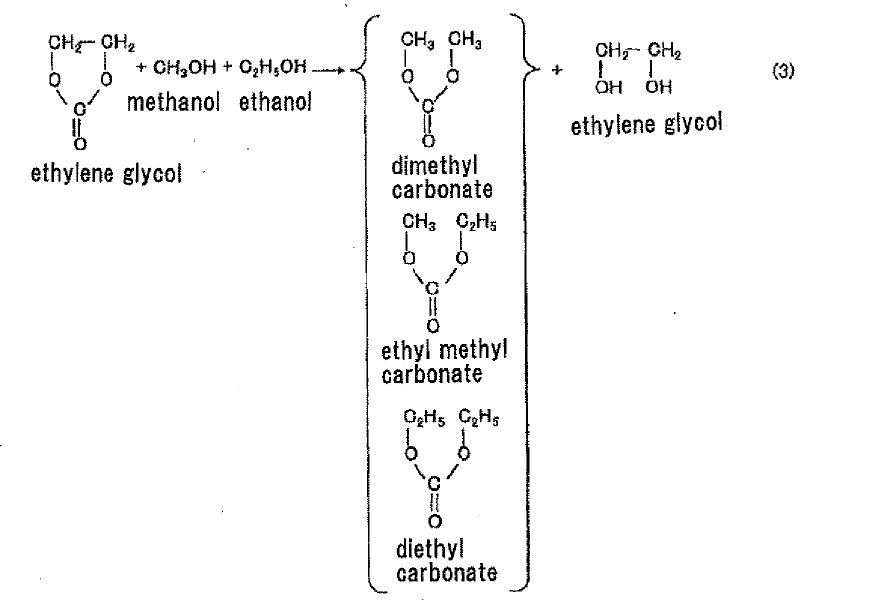

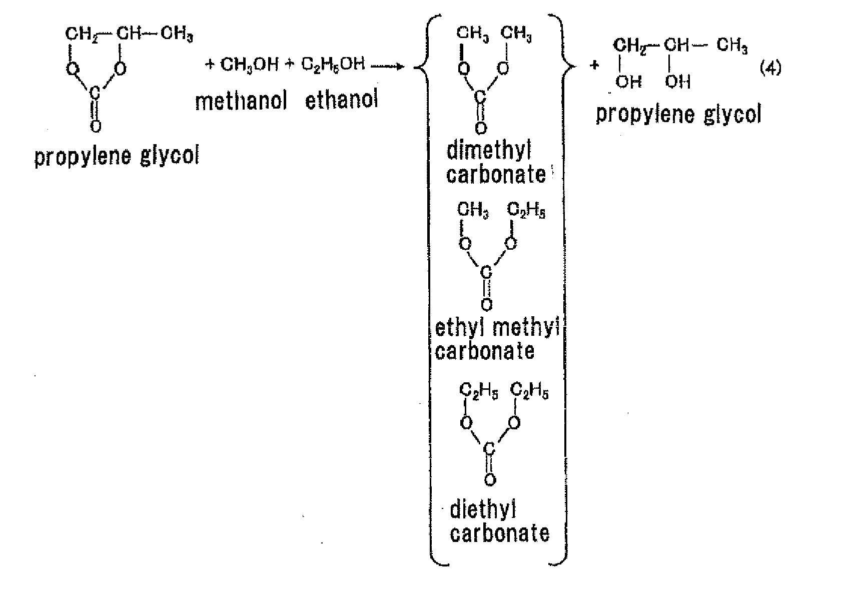

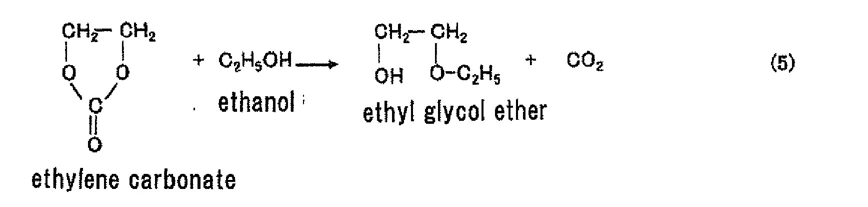

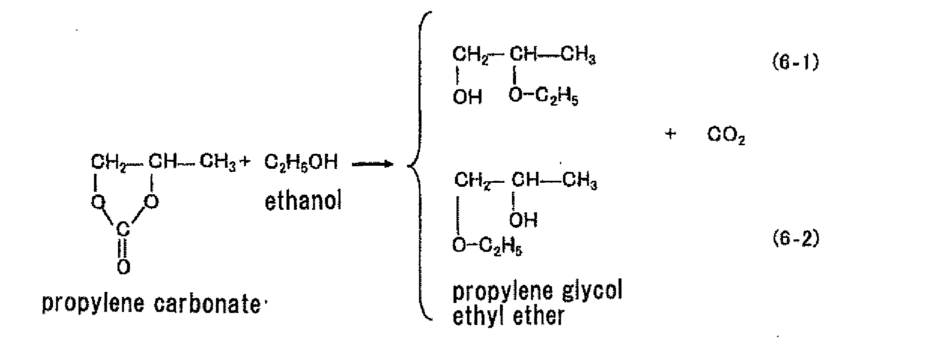

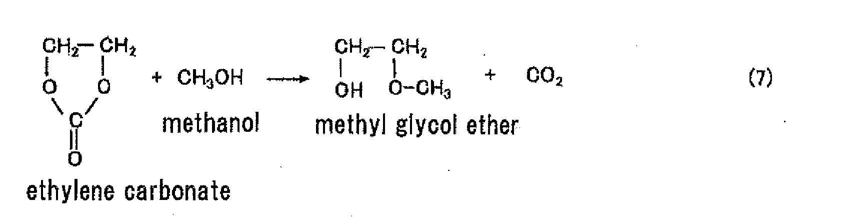

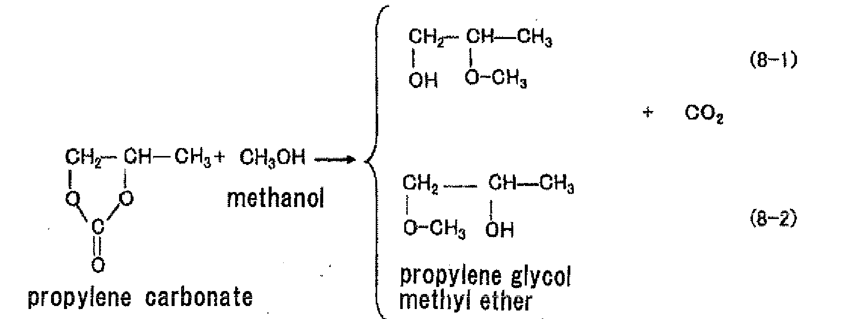

The boiling points of reaction raw materials, target products and the above-described by-products are as follows, and such a by-product is readily azeotroped with the target product. Therefore, it is very difficult to separate by distillation the target products and the by-products from the reaction product containing these by-products.

Ethylene carbonate: 246°C
Propylene carbonate: 242°C
Ethanol: 78.3°C
Methanol: 64.6°C
Diethyl carbonate: 127°C
Dimethyl carbonate: 90°C
Ethyl methyl carbonate: 107°C
Ethyl glycol ether: 136°C
Propylene glycol ethyl ether: 133°C (in case of (6)-2)
Methyl glycol ether: 125°C
Propylene glycol methyl ether: 120°C (in case of (8)-2)
Ethylene glycol: 198°C
Propylene glycol: 188°C

That is, the reaction product obtained by a transesterification reaction of ethylene carbonate with ethanol contains diethyl carbonate (boiling point: 127°C) and ethylene glycol (boiling point: 198°C) as target products, ethylene carbonate (boiling point: 246°C) and ethanol (boiling point: 78.3°C) as remaining reaction raw materials, and ethyl glycol ether (boiling point: 136°C) as a by-product. Out of these materials, diethyl carbonate (boiling point: 127°C) and ethyl glycol ether (boiling point: 136°C) have a similar boiling point and are in an azeotropic relationship with each other, and their separation by distillation is difficult.

Also, the reaction product obtained by a transesterification reaction af propylene carbonate with ethanol contains diethyl carbonate (boiling point: 127°C) and propylene glycol (boiling point: 188°C) as target products, propylene carbonate (boiling point: 242°C) and ethanol (boiling point: 78.3°C) as remaining reaction raw materials, and propylene glycol ethyl ether (boiling point: 133°C, in case of (6)-2) as a by-product. Out of these materials, diethyl carbonate (boiling point: 127°C) and propylene glycol ethyl ether (boiling point: 133°C, in case of (6)-2) have a similar boiling point and are in an azeotropic relationship with each other, and their separation by distillation is difficult.

Furthermore, the reaction product obtained by a transesterification reaction of ethylene carbonate with a mixture of ethanol and methanol contains diethyl carbonate (boiling point: 127°C), dimethyl carbonate (boiling point: 90°C), ethyl methyl carbonate (boiling point: 107°C) and ethylene glycol (boiling point: 198°C) as target products, ethylene carbonate (boiling point: 246°C), ethanol (boiling points 78.3°C) and methanol (boiling point: 64.6°C) as remaining reaction raw materials, and ethyl glycol ether (boiling point: 136°C) and methyl glycol ether (boiling point: 125°C) as by-products. Out of these materials, diethyl carbonate (boiling point: 127°C), ethyl glycol ether (boiling point: 136°C), ethyl methyl carbonate (boiling point: 107°C) and methyl glycol ether (boiling point: 125°C) have a similar boiling point and are in an azeotropic relationship with each other, and their separation by distillation is difficult.

In addition, the reaction product obtained by a transesterification reaction of propylene carbonate with a mixture of ethanol and methanol contains diethyl carbonate (boiling point: 127°C), dimethyl carbonate (boiling point: 90°C), ethyl methyl carbonate (boiling point: 107°C) and propylene glycol (boiling point: 188°C) as target products, propylene carbonate (boiling point: 242°C), ethanol (boiling point: 78.3°C) and methanol (boiling point: 64.6°C) as remaining reaction raw materials, and propylene glycol ethyl ether (boiling point: 133°C, in case of (6)-2) and propylene glycol methyl ether (boiling point: 120°C, in case of(8)-(2)) as by-products. Out of these materials, diethyl carbonate (boiling point: 127°C) and propylene glycol ethyl ether (boiling point: 133°C, in case of (6)-2) have a similar boiling point and are in an azeotropic relationship with each other, and ethyl methyl carbonate (boiling point: 107°C), propylene glycol methyl ether (boiling point: 120°C), diethyl carbonate (boiling point 127°C) and propylene glycol ethyl ether (boiling point: 133°C) have a similar boiling point and are in an azeotropic relationship. In both cases, separation by distillation is difficult.

As the method for separating and removing these by-products, there has been heretofore proposed, for example, in Patent Document 1, a method where methyl glycol ether by-produced by a transesterification reaction of ethylene carbonate and methanol is withdrawn as a side stream from a distillation tower and dimethyl carbonate containing no glycol ether is recovered from the top.

### RELATED ART

### PATENT DOCUMENT

Patent Document 1: An unexamined published Japanese patent application JP-A-2002-371037

### SUMMARY OF THE INVENTION

### PROBLEMS THAT THE INVENTION IS TO SOLVE

However, in Patent Document 1, a method for separating ethyl glycol ether from diethyl carbonate, ethyl methyl carbonate, dimethyl carbonate and the like is not disclosed.

Also, a method for separating and removing methyl glycol ether and ethyl glycol ether from a mixture of dimethyl carbonate, ethyl methyl carbonate and diethyl carbonate, containing these ethers, has not been heretofore proposed.

In this way, separation and removal of by-products are difficult and therefore, it has been conventionally not performed to react, for example, ethylene carbonate or propylene carbonate with a mixture of ethanol and methanol by transesterification in the same reactor.
Also, in the production of diethyl carbonate by a transesterification of ethylene carbonate or propylene carbonate with ethanol, the target product cannot be efficiently produced in high purity, because separation and removal of by-product glycol ethers are difficult.

An object of the present invention is to provide an industrially advantageous process for producing a symmetric dialkyl carbonate and an asymmetric dialkyl carbonate by performing a transesterification reaction of an alkylene carbonate and two or more kinds of alcohols, particularly, the process where a by-product that is azeotropic with a target product is efficiently separated and removed and the target product is produced in high purity with good efficiency.

Another object of the present invention is to provide a process for producing diethyl carbonate by performing a transesterification reaction of ethylene carbonate or propylene carbonate with ethanol, where a by-product that is azeotropic with the target product is efficiently separated and removed and the target product is produced in high purity with good efficiency.

### MEANS FOR SOLVING THE PROBLEMS

As a result of various investigations to solve the above-described problems, the present inventors have found that in the production of a dialkyl carbonate through transesterification between an alkylene carbonate and an alcohol, a by-product that is azeotropic with a target product can be easily separated by distillation from the target product, by performing extractive distillation using, as the extraction solvent, the same alkylene glycol as that produced by the transesterification. The present invention has been accomplished based on this finding.

That is, the gist of the present invention resides in the followings.

[1] A process for producing a symmetric dialkyl carbonate and an asymmetric dialkyl carbonate, comprising performing a transesterification reaction of an alkylene carbonate with two or more kinds of alcohols in a same reactor.

[2] The process for producing a symmetric dialkyl carbonate and an asymmetric dialkyl carbonate as described in [1], wherein the alkylene carbonate is ethylene carbonate, the two or more kinds of alcohols are ethanol and methanol, the symmetric dialkyl carbonate is diethyl carbonate and dimethyl carbonate, and the asymmetric dialkyl carbonate is ethyl methyl carbonate.

[3] The process for producing a symmetric dialkyl carbonate and an asymmetric dialkyl carbonate as described in [1] or [2], wherein the transesterification reaction is performed while removing an ether compound produced by the transesterification reaction, from the transesterification reaction solution.

[4] The process for producing a symmetric dialkyl carbonate and an asymmetric dialkyl carbonate as described in [3], wherein the ether compound is removed by subjecting the transesterification reaction solution to extractive distillation using, as the extraction solvent, the same alkylene glycol as the alkylene glycol produced by the transesterification reaction.

[5] The process for producing a symmetric dialkyl carbonate and an asymmetric dialkyl carbonate as described in [4], wherein the process comprises the following step 1 to step 5 and the extractive distillation is performed in the step 2:
   (1) a step 1 of performing a transesterification reaction of an alkylene carbonate with two or more kinds of alcohols,
   (2) a step 2 of separating by distillation the reaction product in the step 1 into a low-boiling fraction mainly comprising an alcohol, a symmetric dialkyl carbonate and an asymmetric dialkyl carbonate and a high-boiling fraction mainly comprising an alkylene carbonate and an alkylene glycol,
   (3) a step 3 of separating by distillation the low-boiling fraction separated by distillation in the step 2, into a low-boiling fraction mainly comprising an alcohol and a high-boiling fraction mainly comprising a symmetric dialkyl carbonate and an asymmetric dialkyl carbonate,
   (4) a step 4 of separating by distillation the high-boiling fraction separated by distillation in the step 2, into a low-boiling fraction mainly comprising an alkylene glycol and containing an alkylene carbonate and a high-boiling fraction mainly comprising an alkylene carbonate, and
   (5) a step 5 of recycling the low-boiling fraction separated by distillation in the step 3 and the high-boiling fraction separated by distillation in the step 4, to the step 1.

[6] The process for producing a symmetric dialkyl carbonate and an asymmetric dialkyl carbonate as described in [5], wherein the alkylene glycol in the low-boiling fraction separated by distillation in the step 4 is used as the extraction solvent.
[7] The process for producing a symmetric dialkyl carbonate and an asymmetric dialkyl carbonate as described in [5] or [6], wherein the process comprises a step 6 of obtaining an alkylene glycol by hydrolyzing the alkylene carbonate in the low-boiling fraction separated by distillation in the step 4.

[8] The process for producing a symmetric dialkyl carbonate and an asymmetric dialkyl carbonate as described in [4], wherein the alkylene carbonate is ethylene carbonate, the two or more kinds of alcohols are ethanol and methanol, the symmetric dialkyl carbonate is diethyl carbonate and dimethyl carbonate, the asymmetric dialkyl carbonate is ethyl methyl carbonate, the process comprises the following step i to step v, and the extractive distillation is performed in the step ii:
   (i) a step i of performing a transesterification reaction of ethylene carbonate with a mixture of ethanol and methanol,
   (ii) a step ii of separating by distillation the reaction product in the step i into a low-boiling fraction mainly comprising ethanol, methanol, diethyl carbonate, dimethyl carbonate and ethyl methyl carbonate and a high-boiling fraction mainly comprising ethylene carbonate and ethylene glycol,
   (iii) a step iii of separating by distillation the low-boiling fraction separated by distillation in the step ii, into a low-boiling fraction mainly comprising ethanol and methanol and a high-boiling fraction mainly comprising diethyl carbonate, dimethyl carbonate and ethyl methyl carbonate,
   (iv) a step iv of separating by distillation the high-boiling fraction separated by distillation in the step ii, into a low-boiling fraction mainly comprising ethylene glycol and containing ethylene carbonate and a high-boiling fraction mainly comprising ethylene carbonate, and
   (v) a step v of recycling the low-boiling fraction separated by distillation in the step iii and the high-boiling fraction separated by distillation in the step iv, to the step i.

[9] The process for producing a symmetric dialkyl carbonate and an asymmetric dialkyl carbonate as described in [8], wherein the ethylene glycol in the low-boiling fraction separated by distillation in the step iv is used as the extraction solvent.

[10] The process for producing a symmetric dialkyl carbonate and an asymmetric dialkyl carbonate as described in [8] or [9], wherein the process comprises a step vi of obtaining ethylene glycol by hydrolyzing the ethylene carbonate in the low-boiling fraction separated by distillation in the step iv.
[11] A process for producing diethyl carbonate, comprising performing a transesterification reaction of ethylene carbonate or propylene carbonate with ethanol, wherein the process comprises a step of subjecting the reaction product obtained in the transesterification reaction to extractive distillation using ethylene glycol or propylene glycol as the extraction solvent to separate by distillation a fraction containing an ether compound.

[12] The process for producing diethyl carbonate as described in [11], wherein the process comprises the following step I to step V and the extractive distillation is performed in the step II:
   (I) a step I of performing a transesterification reaction of ethylene carbonate or propylene carbonate with ethanol,
   (II) a step II of separating by distillation the reaction product in the step I into a low-boiling fraction mainly comprising ethanol and diethyl carbonate and a high-boiling fraction mainly comprising ethylene carbonate or propylene carbonate and ethylene glycol or propylene glycol,
   (III) a step III of separating by distillation the low-boiling fraction separated by distillation in the step II, into a low-boiling fraction mainly comprising ethanol and a high-boiling fraction mainly comprising diethyl carbonate,
   (IV) a step IV of separating by distillation the high-boiling fraction separated by distillation in the step II, into a low-boiling fraction mainly comprising ethylene glycol or propylene glycol and containing ethylene carbonate or propylene carbonate and a high-boiling fraction mainly comprising ethylene carbonate or propylene carbonate, and
   (V) a step V of recycling the low-boiling fraction separated by distillation in the step III and the high-boiling fraction separated by distillation in the step IV, to the step I.

[13] The process for producing diethyl carbonate as described in [12], wherein the ethylene glycol or propylene glycol in the low-boiling fraction separated by distillation in the step IV is used as the extraction solvent.
[14] The process for producing diethyl carbonate as described in [12] or [13], wherein the process comprises a step VI of obtaining ethylene glycol or propylene glycol by hydrolyzing the ethylene carbonate or propylene carbonate in the low-boiling fraction separated by distillation in the step IV.

### EFFECT OF THE INVENTION

According to the production process of a symmetric dialkyl carbonate and an asymmetric dialkyl carbonate of the present invention, a transesterification reaction of an alkylene carbonate with two or more kinds of alcohols is performed in the same reactor, whereby a symmetric dialkyl carbonate and an asymmetric dialkyl carbonate can be produced efficiently at the same time.

In particular, the transesterification reaction is performed while removing an ether compound produced by the transesterification reaction, from the transesterification reaction solution, more specifically, an ether compound as a by-product is removed by subjecting the transesterification reaction solution to extractive distillation using, as the extraction solvent, the same alkylene glycol as the alkylene glycol produced by the transesterification reaction, whereby a by-product that is azeotropic with a target product can be efficiently separated and removed and the target product can be produced in high purity with good efficiency.

The production process of a symmetric dialkyl carbonate and an asymmetric dialkyl carbonate of the present invention is effective particularly in producing diethyl carbonate and dimethyl carbonate as the symmetric dialkyl carbonate and ethyl methyl carbonate as the asymmetric dialkyl carbonate by using ethylene carbonate as the alkylene carbonate and a mixture of ethanol and methanol as the two or more kinds of alcohols.

That is, in the case of simultaneously producing diethyl carbonate, ethyl methyl carbonate and dimethyl carbonate by performing a transesterification reaction of ethylene carbonate with a mixture of ethanol and methanol, methyl glycol ether and ethyl glycol ether as by-products are in an azeotropic relationship with ethyl methyl carbonate and diethyl carbonate, respectively, and can be hardly separated by normal distillation, as a result, the by-products are unavoidably mixed in ethyl methyl carbonate and diethyl carbonate as target products, which gives rise to reduction in purity of the target product.
In this case, according to the present invention, extractive distillation is performed using ethylene glycol or propylene glycol as the extraction solvent, whereby those by-products can be efficiently separated from the target products and the target products can be produced in high purity with good efficiency.

Also, ethyl glycol ether by-produced when producing diethyl carbonate by performing a transesterification reaction of ethylene carbonate or propylene carbonate with ethanol is in an azeotropic relationship with diethyl carbonate and can be hardly separated by normal distillation, as a result, the by-product is unavoidably mixed in diethyl carbonate as a target product, which gives rise to reduction in purity of the target product. However, according to the production process of diethyl carbonate of the present invention, extractive distillation is performed using ethylene glycol or propylene glycol as the extraction solvent, whereby the by-product can be efficiently separated from the target product and the target product can be produced in high purity with good efficiency.

### BRIEF DESCRIPTION OF THE DRAWINGS

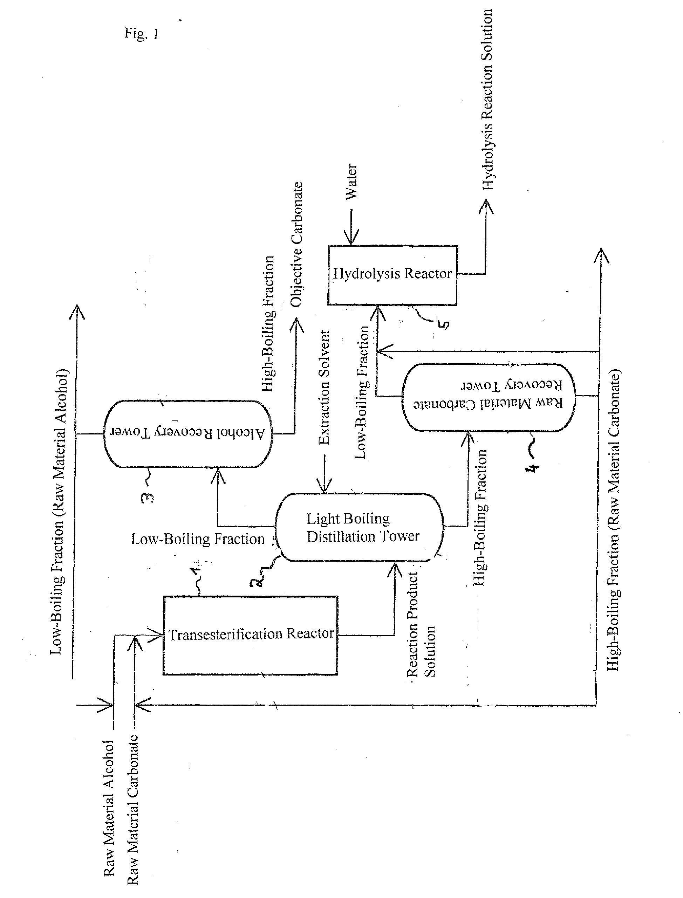

[Fig. 1] A production process view showing one embodiment of the process for producing a dialkyl carbonate according to the present invention.

### MODE FOR CARRYING OUT THE INVENTION

The mode for carrying out the present invention is described in detail below, but the configuration requirements in the following description are one example (representative example) of the embodiment of the present invention and the present invention is not limited to these contents as long as its gist is observed.

In the production process of a symmetric dialkyl carbonate and an asymmetric dialkyl carbonate of the present invention, the alkylene carbonate as a reaction raw material is not particularly limited but includes, for example, an alkylene carbonate containing an alkylene group having a carbon number of 2 to 10, and specific examples thereof include ethylene carbonate, propylene carbonate, butylene carbonate, vinyl ethylene carbonate, cyclohexene carbonate and styrene carbonate.

Two or more of these alkylene carbonates may be used for the transesterification reaction, but usually, one kind of an alkylene carbonate is used.

Out of these alkylene carbonates, considering difficulty in separating by distillation an ether compound as a by-product of the transesterification reaction from the objective dialkyl carbonate, the effects of the present invention are advantageously brought out when ethylene carbonate or propylene carbonate, particularly, ethylene carbonate, is used as the raw material alkylene carbonate.

The alcohol used for the transesterification reaction with the alkylene carbonate is not particularly limited but includes an alcohol having a carbon number of 1 to 10, and specific examples thereof include methanol, ethanol, propanol, isopropanol, butanol and isobutanol. In the production process of a symmetric dialkyl carbonate and an asymmetric dialkyl carbonate of the present invention, arbitrary two or more kinds, preferably two or three kinds, of these alcohols are used for the transesterification reaction.

The combination thereof is also not particularly limited, but out of these alcohols, considering difficulty in separating by distillation an ether compound as a by-product of the transesterification reaction from the objective dialkyl carbonate, the advantages of the present invention are effectively brought out particularly when methanol and ethanol are used as the raw material alcohol.

The production process of a symmetric dialkyl carbonate and an asymmetric dialkyl carbonate of the present invention is effective particularly for a process of producing diethyl carbonate and dimethyl carbonate as the symmetric dialkyl carbonate and ethyl methyl carbonate as the asymmetric dialkyl carbonate by using ethylene carbonate as the alkylene carbonate and a mixture of ethanol and methanol as the two or more kinds of alcohols.

In the transesterification reaction of an alkylene carbonate with two or more kinds of alcohols in the same reactor, the transesterification reaction is preferably performed while removing an ether compound as a by-product of the transesterification reaction from the transesterification reaction solution. The expression "perform the transesterification reaction while removing an ether compound from the transesterification reaction solution" means that the production process includes a step of removing an ether compound in any stage until the step of purifying the symmetric dialkyl carbonate and asymmetric dialkyl carbonate of the present invention (for example, the following step 3). This removal of an ether compound is preferably performed by extractive distillation using, as the extraction solvent, the same alkylene glycol as the alkylene glycol produced by the transesterification reaction. Also, the ether compound may be removed by water extraction by adding water in an amount of 0. 1 to 5 times by weight the total amount of symmetric and asymmetric dialkyl carbonates to the transesterification reaction solution. When water is added and the system is left standing still, the solution is separated into two layers of oil and water and after separating and collecting the oil layer by an appropriate method, a symmetric dialkyl carbonate and an asymmetric dialkyl carbonate can be separated by distillation or the like.

The production process of a symmetric dialkyl carbonate and an asymmetric dialkyl carbonate of the present invention preferably comprises the following step 1 to step 5 and more preferably further contains the following step 6, and the above-described extractive distillation is preferably performed in the step 2:

(1) a step 1 of performing a transesterification reaction of an alkylene carbonate with two or more kinds of alcohols,
(2) a step 2 of separating by distillation the reaction product in the step 1 into a low-boiling fraction mainly comprising an alcohol, a symmetric dialkyl carbonate and an asymmetric dialkyl carbonate (the term "mainly comprising" means to contain in a ratio of 95 wt% or more, preferably 99 wt% or more, based on the entirety) and a high-boiling fraction mainly comprising an alkylene carbonate and an alkylene glycol (the term "mainly comprising" means to contain in a ratio of 95 wt% or more, preferably 99 wt% or more, based on the entirety),
(3) a step 3 of separating by distillation the low-boiling fraction separated by distillation in the step 2, into a low-boiling fraction mainly comprising an alcohol (the term "mainly comprising" means to contain in a ratio of 95 wt% or more, preferably 99 wt% or more, based on the entirety) and a high-boiling fraction mainly comprising a symmetric dialkyl carbonate and an asymmetric dialkyl carbonate (the term "mainly comprising" means to contain in a ratio of 95 wt% or more, preferably 99 wt% or more, based on the entirety),
(4) a step 4 of separating by distillation the high-boiling fraction separated by distillation in the step 2, into a low-boiling fraction mainly comprising an alkylene glycol (the term "mainly comprising" as used herein means to contain in a ratio of 80 wt% or more, preferably 85 wt% or more, based on the entirety) and containing an alkylene carbonate and a high-boiling fraction mainly comprising an alkylene carbonate (the term "mainly comprising" means to contain in a ratio of 95 wt% or more, preferably 99 wt% or more, based on the entirety),
(5) a step 5 of recycling the low-boiling fraction separated by distillation in the step 3 and the high-boiling fraction separated by distillation in the step 4, to the step 1, and
(6) a step 6 of obtaining an alkylene glycol by hydrolyzing the alkylene carbonate in the low-boiling fraction separated by distillation in the step 4.

As described above, the production process of a symmetric dialkyl carbonate and an asymmetric dialkyl carbonate of the present invention is effective particularly for a process of producing diethyl carbonate and dimethyl carbonate as the symmetric dialkyl carbonate and ethyl methyl carbonate as the asymmetric dialkyl carbonate by using ethylene carbonate as the alkylene carbonate and a mixture of ethanol and methanol as the two or more kinds of alcohols. In this case, the production process of a symmetric dialkyl carbonate and an asymmetric dialkyl carbonate of the present invention preferably comprises the following step i to step v and more preferably further contains the following step vi, and the above-described extractive distillation is preferably performed in the step ii:

(i) a step i of performing a transesterification reaction of ethylene carbonate with a mixture of ethanol and methanol,
(ii) a step ii of separating by distillation the reaction product in the step i into a low-boiling fraction mainly comprising ethanol, methanol, diethyl carbonate, dimethyl carbonate and ethyl methyl carbonate (the term "mainly comprising" means to contain in a ratio of 95 wt% or more, preferably 99 wt% or more, based on the entirety) and a high-boiling fraction mainly comprising ethylene carbonate and ethylene glycol (the term "mainly comprising" means to contain in a ratio of 95 wt% or more, preferably 99 wt% or more, based on the entirety),
(iii) a step iii of separating by distillation the low-boiling fraction separated by distillation in the step ii, into a low-boiling fraction mainly comprising ethanol and methanol (the term "mainly comprising" means to contain in a ratio of 95 wt% or more, preferably 99 wt% or more, based on the entirety) and a high-boiling fraction mainly comprising diethyl carbonate, dimethyl carbonate and ethyl methyl carbonate (the term "mainly comprising" means to contain in a ratio of 95 wt% or more, preferably 99 wt% or more, based on the entirety),
(iv) a step iv of separating by distillation the high-boiling fraction separated by distillation in the step ii, into a low-boiling fraction mainly comprising ethylene glycol (the term "mainly comprising" as used herein means to contain in a ratio of 80 wt% or more, preferably 85 wt% or more, based on the entirety) and containing ethylene carbonate and a high-boiling fraction mainly comprising ethylene carbonate (the term "mainly comprising" means to contain in a ratio of 95 wt% or more, preferably 99 wt% or more, based on the entirety),
(v) a step v of recycling the low-boiling fraction separated by distillation in the step iii and the high-boiling fraction separated by distillation in the step iv, to the step i, and
(vi) a step vi of obtaining ethylene glycol by hydrolyzing the ethylene carbonate in the low-boiling fraction separated by distillation in the step iv.

The production process of diethyl carbonate of the present invention is a process for producing diethyl carbonate by performing a transesterification reaction of ethylene carbonate or propylene carbonate with ethanol, wherein the process comprises a step of subjecting the reaction product obtained in the transesterification reaction to extractive distillation using ethylene glycol or propylene glycol as the extraction solvent to separate by distillation a fraction containing an ether compound.

The production process of diethyl carbonate of the present invention preferably comprises the following step I to step V and more preferably further contains the following step VI, and the extractive distillation is preferably performed in the step II:
(I) a step I of performing a transesterification reaction of ethylene carbonate or propylene carbonate with ethanol,
(II) a step II of separating by distillation the reaction product in the step I into a low-boiling fraction mainly comprising ethanol and diethyl carbonate (the term "mainly comprising" means to contain in a ratio of 95 wt% or more, preferably 99 wt% or more, based on the entirety) and a high-boiling fraction mainly comprising ethylene carbonate or propylene carbonate and ethylene glycol or propylene glycol (the term "mainly comprising" means to contain in a ratio of 95 wt% or more, preferably 99 wt% or more, based on the entirety),
(III) a step III of separating by distillation the low-boiling fraction separated by distillation in the step II, into a low-boiling fraction mainly comprising ethanol (the term "mainly comprising" means to contain in a ratio of 95 wt% or more, preferably 99 wt% or more, based on the entirety) and a high-boiling fraction mainly comprising diethyl carbonate (the term "mainly comprising" means to contain in a ratio of 95 wt% or more, preferably 99 wt% or more, based on the entirety),
(IV) a step IV of separating by distillation the high-boiling fraction separated by distillation in the step II, into a low-boiling fraction mainly comprising ethylene glycol or propylene glycol (the term "mainly comprising" as used herein means to contain in a ratio of 80 wt% or more, preferably 85 wt% or more, based on the entirety) and containing ethylene carbonate or propylene carbonate and a high-boiling fraction mainly comprising ethylene carbonate or propylene carbonate (the term "mainly comprising" means to contain in a ratio of 95 wt% or more, preferably 99 wt% or more, based on the entirety),
(V) a step V of recycling the low-boiling fraction separated by distillation in the step III and the high-boiling fraction separated by distillation in the step IV, to the step I, and
(VI) a step VI of obtaining ethylene glycol or propylene glycol by hydrolyzing the ethylene carbonate or propylene carbonate in the low-boiling fraction separated by distillation in the step IV.

The present invention is described in detail below by referring to Fig. 1 showing one embodiment of the production step in the production process of a symmetric dialkyl carbonate and an asymmetric dialkyl carbonate or the production process of diethyl carbonate of the present invention, but the present invention is not limited to the process shown in Fig. 1 by any means. Also, in the following, the production process of a symmetric dialkyl carbonate and an asymmetric dialkyl carbonate of the present invention, where ethylene carbonate or propylene carbonate is used as the alkylene carbonate and a mixture of ethanol and methanol is used as the two or more kinds of alcohols, is described by way of example, but the kind of the alkylene carbonate and the kind of the alcohol are not limited thereto.

Incidentally, in the following, ethylene carbonate or propylene carbonate is sometimes simply referred to as a "raw material carbonate", and ethanol or a mixture of ethanol and methanol is sometimes simply referred to as a "raw material alcohol,".
Furthermore, diethyl carbonate, dimethyl carbonate and ethyl methyl carbonate, which are a target product produced in the main reaction, are sometimes referred to as an "objective carbonate"; ethylene glycol or propylene glycol is sometimes referred to as a "product glycol"; and ethyl glycol ether, methyl glycol ether, propylene glycol ethyl ether and propylene glycol methyl ether, which are a by-product, are sometimes referred to as a "by-product glycol ether".

In the process of Fig. 1, a raw material carbonate (ethylene carbonate or propylene carbonate) and a raw material alcohol (ethanol or a mixture of ethanol and methanol) are introduced into a transesterification reactor 1 together with the low-boiling fraction from the later-described alcohol recovery tower 3 and the high-boiling fraction from the later-described raw material carbonate recovery tower 4 to perform a transesterification reaction (step 1, step i, step I).

In any process, the transesterification reaction is performed in the presence of a catalyst. As the catalyst used here, a known catalyst generally used as a transesterification reaction catalyst can be appropriately selected and used. In the case of a homogeneous catalyst, for example, amines such as triethylamine, alkali metals such as sodium, alkali metal compounds such as sodium chloroacetate and sodium methylate, and thallium compounds are used. In the case of a heterogeneous catalyst, for example, ionexchange resins modified with a functional group, amorphous silicas impregnated with silicate of an alkali metal or an alkaline earth metal, ammonium-exchanged Y-type zeolite, and a mixed oxide of cobalt and nickel are used. One of these catalysts may be used alone, or two or more thereof may be used in combination.

The transesterification reaction catalyst is preferably a heterogeneous catalyst requiring no separation operation from the reaction product. Specifically, an ionexchange resin or the like is used, and a gel-type strongly basic anion-exchange resin is preferably used.

The reactor for performing the transesterification reaction may be either a batch-type reactor or a fixed bed reactor. The reaction conditions can be appropriately selected according to the reactor, raw materials (raw material carbonate, raw material alcohol) and catalyst used, but, for example, the reaction temperature is from 40 to 200°C, the mixing ratio of raw materials is from 0.1 to 20 in terms of molar ratio of the raw material alcohol to the raw material carbonate, the reaction pressure is from 10 to 2,000 kPa, and the reaction is performed for 0.5 to 10 hours.

This transesterification reaction is an equilibrium reaction and therefore, the reaction product contains the raw material carbonate and raw material alcohol in addition to the objective carbonate (dimethyl carbonate, ethyl methyl carbonate, diethyl carbonate), which is the target product, and a product glycol (ethylene glycol or propylene glycol). Furthermore, due to a side reaction occurring simultaneously with the main reaction, ethyl glycol ether is by-produced in case of reaction between ethylene carbonate and ethanol, propylene glycol ethyl ether is by-produced in case of reaction between propylene carbonate and ethanol, methyl glycol ether is by-produced in case of reaction between ethylene carbonate and methanol, and propylene glycol methyl ether is by-produced in case of reaction between propylene carbonate and methanol, and these glycol ethers are contained in respective reaction product solutions.
As described above, such a by-product glycol ether is close in the boiling point to the objective carbonate and can be hardly separated by distillation.

Incidentally, hydroxyethyl methyl carbonate and hydroxyethyl ethyl carbonate (hereinafter, these are sometimes referred to as a "hydroxycarbonate") as intermediates of the main reaction are present in the reaction product solution, but these intermediates are decomposed into a raw material carbonate and a raw material alcohol by heating in the later distillative separation step and circulated to the reaction step as an unreacted raw material and therefore, their mixing in the target product does not become a problem.

The reaction product solution in the transesterification reactor 1 is sent to a light boiling distillation tower 2 and separated by distillation into a low-boiling fraction mainly comprising a raw material alcohol and an objective carbonate (the term "mainly comprising" means to contain in a ratio of 95 wt% or more, preferably 99 wt% or more, based on the entirety) and a high-boiling fraction mainly comprising a raw material carbonate and a product glycol (the term "mainly comprising" means to contain in a ratio of 95 wt% or more, preferably 99 wt% or more, based on the entirety) (step 2, step ii, step II).

During distillation in the light boiling distillation tower 2, the above-described hydroxycarbonate as a reaction intermediate is decomposed by heating into a raw material carbonate and a raw material alcohol.

As described above, the reaction product solution obtained by the transesterification reaction contains an objective carbonate as the target product and a by-product glycol ether which can be hardly separated by distillation. In the present invention, this by-product glycol ether is separated from the objective carbonate by performing extractive distillation using, as the extraction solvent, ethylene glycol when ethylene carbonate is used as the raw material carbonate, or propyleneglycol when propylene carbonate is used as the raw material carbonate.

That is, the by-product glycol ether is withdrawn from the bottom of the light boiling distillation tower 2 of Fig. 1 as a high-boiling fraction together with ethylene glycol or propylene glycol as the extraction solvent and the raw material carbonate, whereby the by-product glycol ether and the objective carbonate can be efficiently separated by distillation.

In the present invention, the distillation tower for performing such extractive distillation is not particularly limited, but in the case of passing through the production steps shown in Fig. 1, the extractive distillation is preferably performed in this light boiling distillation tower 2.
Accordingly, the extraction solvent is supplied to the light boiling distillation tower 2 together with the reaction product solution from the transesterification reactor 1 and at the separation by distillation in the light boiling distillation tower 2 into a low-boiling fraction containing a raw material alcohol and an objective carbonate and a high-boiling fraction containing a raw material carbonate and a product glycol, the by-product glycol ether is transferred into the high-boiling fraction and effectively separated by distillation from the objective carbonate. In a raw material carbonate recovery tower 4, the separated by-product glycol ether in the high-boiling fraction is separated to the low-boiling fraction side mainly comprising a product glycol (the term "mainly comprising" as used herein means to contain in a ratio of 80 wt% or more, preferably 85 wt% or more, based on the entirety) and containing a raw material carbonate. The separated by-product glycol ether in this low-boiling fraction is separated and removed together with water from the product glycol during dehydration after passing through a hydrolysis reactor 5 in the later stage.

In the present invention, as the distillation tower for performing the extractive distillation (in Fig. 1, the light boiling distillation tower 2), a tower of any type, for example, a packed tower, a packed tower packed with a regular packing material such as Sulzer packing, Mellapack and MC Pack or an irregular packing material such as IMTP and Raschig ring, a bubble-cap tower, or a tray tower using a sieve tray or a bubble tray, may be used.

The supply amount and supply rate of the extraction solvent and the supplied portion of the extractive distillation tower may be appropriately selected according to the composition or supply amount of the transesterification reaction product solution, but, for example, the following conditions are preferably employed.

### <In case of using ethylene glycol as the extraction solvent>

Supply amount: From 0.1 to 10 times by weight the feed amount of the distillation raw material.
Supplied portion: The ethylene glycol-supplied portion is not particularly limited as long as it is the same as the raw material-supplied portion or higher than that of the extractive distillation tower, but if supplied to the top, the extraction solvent is disadvantageously mixed with the distillate liquid. Accordingly, the supplied portion is preferably the upper part of the tower.

### <In case of using propylene glycol as the extraction solvent>

Supply amount: From 0.1 to 10 times by weight the feed amount of the distillation raw material.
Supplied portion: The propylene glycol-supplied portion is not particularly limited as long as it is the same as the raw material-supplied portion or higher than that of the extractive distillation tower, but if supplied to the top, the extraction solvent is disadvantageously mixed with the distillate liquid. Accordingly, the supplied portion is preferably the upper part of the tower.

If the supply amount of the extraction solvent is too smaller than the range above, the effects of the present invention produced by use of the extraction solvent cannot be sufficiently obtained, whereas if it is excessively large, the energy for recovery of the extraction solvent becomes large. Also, when the extraction solvent-supplied portion is higher than the raw material-supplied portion, the extraction effect is increased, and if it is the top, the extraction solvent is readily mixed with the distillate liquid and this is not preferred.

The conditions of this extractive distillation are appropriately determined according to the composition of raw materials, but the extractive distillation is preferably performed, for example, under a pressure of 1 to 100 kPa at a reflux ratio of approximately from 0.01 to 10. As for the extraction solvent, a solvent obtained outside the production process in the process for producing a symmetric dialkyl carbonate, an asymmetric dialkyl carbonate or diethyl carbonate of the present invention may be used, but, for example, a low-boiling fraction may be withdrawn as a side stream from the upper part of the later-described raw material carbonate recovery tower 4 and used as the extraction solvent. Here, the position at which the low-boiling fraction is withdrawn is a position being located higher than the feed solution-supplied portion of the raw material carbonate recovery tower 4 and giving a by-product glycol ether concentration of 100 ppm by weight or less, preferably 50 ppm by weight or less, to the low-boiling fraction withdrawn.

The low-boiling fraction separated by distillation in the light boiling distillation tower 2 contains a raw material alcohol and an objective carbonate and does not substantially contain by-product glycol ether. The term " does not substantially contains" means that the raw material alcohol and the objective carbonate account for 95 wt% or more, preferably 99 wt% or more, based on the entirety. This low-boiling fraction is then sent to an alcohol recovery tower 3 and separated by distillation into a low-boiling fraction mainly comprising the raw material alcohol (the term "mainly comprising" means to contain in a ratio of 95 wt% or more, preferably 99 wt% or more, based on the entirety) and a high-boiling fraction mainly comprising an objective carbonate (the term "mainly comprising" means to contain in a ratio of 95 wt% or more, preferably 99 wt% or more, based on the entirety) (step 3, step iii, step III).

The high-boiling fraction mainly comprising an objective carbonate is, if desired, further purified to make a finished product. For example, in the case of containing diethyl carbonate, dimethyl carbonate and ethyl methyl carbonate as objective carbonates, these are sequentially distilled, whereby each objective carbonate can be recovered as a high-purity component.

On the other hand, the high-boiling fraction separated by distillation in the light boiling distillation tower 2 is sent to a raw material carbonate recovery tower 4 and separated by distillation into a low-boiling fraction which is an azeotropic mixture mainly comprising a product glycol (the term "mainly comprising" means to contain in a ratio of 80 wt% or more, preferably 85 wt% or more, based on the entirety) and containing a raw material carbonate and a by-product glycol ether and a high-boiling fraction mainly comprising a raw material carbonate (the term "mainly comprising" means to contain in a ratio of 95 wt% or more, preferably 99 wt% or more, based on the entirety) (step 4, step iv, step IV).

The high-boiling fraction being separated by distillation in the raw material carbonate recovery tower 4 and mainly comprising a raw material carbonate and the low-boiling fraction being separated by distillation in the alcohol recovery tower 3 and mainly comprising a raw material alcohol are returned to the transesterification reactor 1 and recycled as a raw material carbonate and a raw material alcohol, respectively (step 5, step v, step V).

Also, the low-boiling fraction mainly comprising a product glycol and containing a raw material carbonate and a by-product glycol ether, which is separated by distillation in the raw material carbonate recovery tower 4, is sent to a hydrolysis reactor 5, and water is added to the raw material carbonate in the low-boiling fraction to hydrolyze the raw material carbonate in the presence of a catalyst and obtain a product glycol (ethylene glycol or propylene glycol), whereby a reaction product solution mainly comprising a product glycol is obtained (step 6, step vi, step VI). This hydrolysis reaction product solution is dehydrated and purified by distillation or the like in a conventional manner, and ethylene glycol or propylene glycol as a product is withdrawn. At this time, the by-product glycol ether has a boiling point sufficiently lower than that of ethylene glycol or propylene glycol and therefore, efficiently separated and removed to the water side. Incidentally, the high-boiling fraction of the raw material carbonate recovery tower may be purged or may be partially supplied to the hydrolysis reactor for carbonate recovery. Also, the product glycol obtained here may be used as the extraction solvent.

In the present invention, the reaction product solution is subjected to extractive distillation using ethylene glycol or propylene glycol as the extraction solvent in this way, and the by-product glycol ether that is substantially azeotropic with an objective carbonate is efficiently separated by distillation from the objective carbonate, where ethylene glycol or propylene glycol used as the extraction solvent is a glycol present as a by-product glycol in the reaction product solution.
With respect to the reaction product solution contains a by-product glycol in such an amount, ethylene glycol or propylene glycol is supplied as the extraction solvent to the extractive distillation tower, as a result, thanks to an operation mechanism of increasing the relative volatility between glycol ether and the objective carbonate by utilizing affinity of the extraction solvent for glycol ether, good separation effect of the by-product glycol ether can be obtained.

### EXAMPLES

The present invention is described in greater detail below by referring to Examples and Comparative Examples.

### [Transesterification Reaction Using Ethylene Carbonate as Raw Material Carbonate]

### <Adjustment of Catalyst>

A jacket-attached tubular reactor having an inner diameter of 17 mm and a length of 50 cm was used as the transesterification reactor, and 50 mL of strongly basic ionexchange resin (SA-11A, produced by Mitsubishi Chemical Corp.) as a catalyst was packed in the inside. In this reactor, 500 mL of methanol was flowed at 100 mL/hr to wash the resin, and 250 mL of pure water was then flowed at 100 mL/hr to wash the resin with water. Thereafter, 500 mL of an aqueous 2 N-NaOH solution was flowed at 100 mL/hr to convert the resin from CL type to OH type, and 500 mL of pure water was further flowed at 100 mL/hr to remove the aqueous NaOH solution. Finally, methanol was flowed at 100 mL/hr until water in the resin was eliminated.

### <Transesterification Reaction>

The transesterification reactor after adjustment of catalyst above was kept at a temperature of 60°C externally by the jacket, and a mixed raw material of ethylene carbonate, methanol and ethanol (compositional ratio: 1/1/1 (by mol)) was introduced. The reaction was performed at a pressure of 101.3 kPa for a residence time of 2 hours (SV=0.5 hr⁻¹) to obtain a reaction product solution having the following composition.

| (Composition of Reaction Product Solution) | | |
|---|---|---|
| | Dimethyl carbonate | : 7.5 wt% |
| | Ethyl methyl carbonate | : 8.5 wt% |
| | Diethyl carbonate | : 2.1 wt% |
| | Methyl glycol ether | : 0.007 wt% |
| | Ethyl glycol ether | : 0.002 wt% |
| | Ethylene glycol | : 11.4 wt% |
| | Hydroxyethyl methyl carbonate | : 5.8 wt% |
| | Hydroxyethyl ethyl carbonate | : 7.2 wt% |
| | Ethylene carbonate | : 27.9 wt% |
| | Methanol | : 9.7 wt% |
| | Ethanol | : 19.9 wt% |

### <Setting of Model Solution>

In the reaction product solution obtained by the transesterification reaction above, hydroxyethyl methyl carbonate and hydroxyethyl ethyl carbonate as reaction intermediates are present, but these intermediates are decomposed into ethylene carbonate and methanol and into ethylene carbonate and ethanol, respectively, by heating in the distillation tower. Therefore, in the following distillation test of Examples 1 to 3 and Comparative Examples 1 and 2, by taking into consideration the decomposition of the intermediates, Model Solution A having the following composition was used as the distillation raw material and subjected to separation by distillation.

| (Composition of Model Solution A) | | |
|---|---|---|
| | Dimethyl carbonate | : 7.5 wt% |
| | Ethyl methyl carbonate | : 8.5 wt% |
| | Diethyl carbonate | : 2.1 wt% |
| | Methyl glycol ether | : 0.01 wt% |
| | Ethyl glycol ether | : 0.01 wt% |
| | Ethylene glycol | : 11.4 wt% |
| | Ethylene carbonate | : 36.8 wt% |
| | Methanol | : 11.3 wt% |
| | Ethanol | : 22.3 wt% |

### <Example 1>

A glass-made distillation tower having an inner diameter of 40 mm was installed above a 1 L-volume flask and fixed with a column of 600 mm (corresponding to a theoretical tray number of about 12) having packed therein a stainless steel-made packing material (coil pack, 3 mm in diameter), and Model Solution A was fed to the middle of the column.
Under the conditions of a pressure of 13.4 kPa and a reflux ratio of 1, Model Solution A as the distillation raw material was fed at 200 mL/hr and at the same time, ethylene glycol as the extraction solvent was fed at 25 mL/hr from the uppermost part of the column. The distillate liquid from the top and the bottom liquid in the flask at the bottom were analyzed, as a result, the distillate liquid from the top contained 0.01 wt% of methyl glycol ether together with a dialkyl carbonate and an unreacted alcohol, but the concentration of ethyl glycol ether was the determination limit of 10 ppm or less.

### <Example 2>

Extractive distillation was performed under the same conditions as in Example 1 except that the feed amount of ethylene glycol as the extracting agent was changed to 50 mL/hr, as a result, the concentration of both methyl glycol ether and ethyl glycol ether in the distillate liquid from the top was the determination limit or less.

### <Example 3>

Extractive distillation was performed under the same conditions as in Example 1 except that the feed amount of ethylene glycol as the extracting agent was changed to 100 mL/hr, as a result, the concentration of both methyl glycol ether and ethyl glycol ether in the distillate liquid from the top was the determination limit or less.

### <Comparative Example 1>

Extractive distillation was performed under the same conditions as in Example 1 except that the extraction solvent was not used and a column of 800 mm (corresponding to a theoretical tray number of about 16) having packed therein a stainless steel-made packing material (coil pack, 3 mm in diameter) was used in place of the column of 600 mm, as a result, mixing of both methyl glycol ether and ethyl glycol ether in the top distillate liquid was recognized.

### <Comparative Example 2>

In a test of using ethylene glycol as the extraction solvent, diethyl carbonate was not entirely distilled out but mixed in the bottom stream in a small amount. Accordingly, in Comparative Example 2, the extraction solvent was not added, and calculation by a simulation soft was performed for the case of withdrawing a small amount of diethyl carbonate as a bottom liquid, as a result, when the extraction solvent was not used, it was estimated that both methyl glycol ether and ethyl glycol ether are mixed in the top distillate liquid.

Operation conditions and analysis results of Examples 1 to 3 and Comparative Examples 1 and 2 are shown in Tables 1 and 2. Also, the calculation results of Comparative Example 2 are shown in Table 3.

**[Table 1]**

| <Operation Conditions> | | | | | | |
|---|---|---|---|---|---|---|
| | Unit | Example 1 | Example 2 | Example 3 | Comparative Example 1 | Comparative Example 2 |
| Pressure | kPa | 13.4 | 13.4 | 13.4 | 13.4 | 13.4 |
| Theoretical tray number | trays | 12 | 12 | 12 | 16 | 12 |
| Packing material | | coil pack (3 mmφ) | coil pack (3 mmφ) | coil pack (3 mmφ) | coil pack (3 mmφ) | - |
| Packing height | mm | 600 | 600 | 600 | 800 | - |
| Reflux ratio | | 1 | 1 | 1 | 1 | 1 |
| Feed amount of distillation raw material | mL/hr | 200 | 200 | 200 | 200 | 200 |
| Feed amount of ethylene glycol | mL/hr | 25 | 50 | 100 | - | - |

**[Table 2]**

| <Analysis Results> | | | | | |
|---|---|---|---|---|---|
| | | Example 1 | Example 2 | Example 3 | Comparative Example 1 |
| Top distillate liquid (wt%) | methyl glycol ether | 0.01 | <0.001 | <0.001 | 0.01 |
| | ethyl glycol ether | <0.001 | <0.001 | <0.001 | 0.01 |
| Bottom liquid (wt%) | methyl glycol ether | <0.001 | 0.01 | 0.01 | <0.001 |
| | ethyl glycol ether | 0.01 | 0.01 | 0.01 | <0.001 |

**[Table 3]**

| < Calculation Results > | | | | | |
|---|---|---|---|---|---|
| | | Distillation Raw Material | Extraction Solvent | Top Distillate Liquid | Bottom Liquid |
| Comparative Example 2 (wt%) | dimethyl carbonate | 7.3 | | 14.1 | |
| | ethyl methyl carbonate | 8.7 | | 16.8 | |
| | diethyl carbonate | 3.6 | | 6.6 | 0.4 |
| | methyl glycol ether | 0.01 | | 0.019 | <0.001 |
| | ethyl glycol ether | 0.01 | | 0.005 | 0.016 |
| | Ethylene glycol | 12.1 | 0 | | 25.1 |
| | Ethylene carbonate | 35.9 | | | 74.5 |
| | methanol | 11.4 | | 22 | |
| | Ethanol | 21 | | 40.5 | |

### [Transesterification Reaction Using Propylene Carbonate as Raw Material Carbonate]

### <Setting of Model Solution>

A transesterification reaction when using propylene carbonate in place of ethylene carbonate in the transesterification reaction above using ethylene carbonate was calculated by a simulation soft. In the case of propylene carbonate, ethylene glycol is replaced by propylene glycol. Also, methyl glycol ether and ethyl glycol ether as by-products are replaced by propylene glycol methyl ether and propylene glycol ethyl ether, respectively.
Accordingly, in Example 4 and Comparative Example 3, separation by distillation was performed using Model Solution B having the following composition as the distillation raw material.

| (Composition of Model Solution B) | | |
|---|---|---|
| | Dimethyl carbonate | : 16.7 wt% |
| | Ethyl methyl carbonate | : 8.4 wt% |
| | Diethyl carbonate | : 1.4 wt% |
| | Propylene glycol methyl ether | : 0.02 wt% |
| | Propylene glycol ethyl ether | : 0.002 wt% |
| | Propylene glycol | : 10.9 wt% |
| | Propylene carbonate | : 34.2 wt% |
| | Methanol | : 16.3 wt% |
| | Ethanol | : 12.2 wt% |

### <Example 4>

Model Solution B was fed at a flow rate of 2,918 kg/hr to the twelfth tray of a distillation tower having a theoretical tray number of 25 designed in the same manner as in Example 1. Under the conditions of a pressure of 80 mmHg (10.7 kPa) and a reflux ratio of 0.3, extractive distillation of feeding propylene glycol as the extraction solvent at 300 kg/hr to the fourth tray of the distillation tower was performed. As a result, propylene glycol methyl ether and propylene glycol ethyl ether were contained only slightly in the distillate liquid from the top and mostly removed to the bottom.

### <Comparative Example 3>

Distillation was performed under the same conditions as in Example 4 except that the extraction solvent was not used and the reflux ratio was changed to 1.0, as a result, mixing of propylene glycol ethers in the top distillate liquid was recognized.

Operation conditions of Example 4 and Comparative Example 3 are shown in Table 4, and the mass balances are shown in Table 5.

**[Table 4]**

| <Operation Conditions> | | | |
|---|---|---|---|
| | Unit | Example 4 | Comparative Example 3 |
| Pressure | kPa | 10 | 10 |
| Theoretical tray number | | 25 | 25 |
| Tray to which distillation raw material was fed | | 12 | 12 |
| Tray to which extraction solvent was fed | | 4 | - |
| Reflux ratio | | 0.3 3 | 1.0 |
| Feed amount of distillation raw material | kg/hr | 2918 | 2918 |
| Feed amount of propylene glycol | kg/hr | 300 | - |

**[Table 5]**

| <Mass Balance> | | | | | |
|---|---|---|---|---|---|
| | | Feed | Extraction Solvent | Top Distillate Liquid | Bottom Liquid |
| Example 4 (kg/hr) | dimethyl carbonate | 486 | | 486 | |
| | ethyl methyl carbonate | 245 | | 245 | |
| | diethyl carbonate | 40 | | 35 5 | |
| | propylene glycol methyl ether | 0.47 | | 0.005 | 0.465 |
| | propylene glycol ethyl ether | 0.064 | | | 0.064 |
| | propylene glycol | 318 | 300 | | 618 |
| | propylene carbonate | 997 | | | 997 |
| | methanol | 476 | | 476 | |
| | ethanol | 355 | | 355 | |
| Comparative Example 3 (kg/hr) | dimethyl carbonate | 486 | | 486 | |
| | ethyl methyl carbonate | 245 | | 245 | |
| | diethyl carbonate | 40 | | 35 | 5 |
| | propylene glycol methyl ether | 0.47 | | 0.47 | |
| | propylene glycol ethyl ether | 0.064 | | 0.004 | 0.059 |
| | propylene glycol | 318 | 0 | | 318 |
| | propylene carbonate | 997 | | | 997 |
| | methanol | 476 | | 476 | |
| | Ethanol | 355 | | 355 | |

As seen from these results, according to the present invention, glycol ethers which are a by-product in an azeotropic relationship with a dialkyl carbonate such as diethyl carbonate as the target product can be efficiently separated by distillation from the target product by performing extractive distillation using ethylene glycol or propylene glycol as the extraction solvent.

While the invention has been described in detail and with reference to specific embodiments thereof, it will be apparent to one skilled in the art that various changes and modifications can be made therein without departing from the spirit and scope of the invention. This application is based on Japanese Patent Application (Patent Application No. 2008-333601) filed on December 26, 2008 and Japanese Patent Application (Patent Application No. 2009-290722) filed on December 22, 2009, the contents of which are incorporated herein by way of reference.

### INDUSTRIAL APPLICABILITY

According to the present invention, in producing symmetric and asymmetric dialkyl carbonates by performing a transesterification reaction of an alkylene carbonate with two or more kinds of alcohols in the same reactor, by-product glycol ethers can be efficiently separated from the target product, and symmetric and asymmetric dialkyl carbonates can be simultaneously produced in high purity with good efficiency.

### DESCRIPTION OF REFERENCE NUMERALS AND SIGNS

- 1: Transesterification reactor
- 2: Light boiling distillation tower
- 3: Alcohol recovery tower
- 4: Raw material carbonate recovery tower
- 5: Hydrolysis reactor

## Claims

1. A process for producing a symmetric dialkyl carbonate and an asymmetric dialkyl carbonate, comprising performing a transesterification reaction of an alkylene carbonate with two or more kinds of alcohols in a same reactor.

2. The process for producing a symmetric dialkyl carbonate and an asymmetric dialkyl carbonate according to claim 1, wherein said alkylene carbonate is ethylene carbonate, said two or more kinds of alcohols are ethanol and methanol, said symmetric dialkyl carbonate is diethyl carbonate and dimethyl carbonate, and said asymmetric dialkyl carbonate is ethyl methyl carbonate.

3. The process for producing a symmetric dialkyl carbonate and an asymmetric dialkyl carbonate according to claim 1 or 2, wherein said transesterification reaction is performed while removing an ether compound produced by the transesterification reaction, from the transesterification reaction solution.

4. The process for producing a symmetric dialkyl carbonate and an asymmetric dialkyl carbonate according to claim 3, wherein said ether compound is removed by subjecting said transesterification reaction solution to extractive distillation using, as the extraction solvent, the same alkylene glycol as the alkylene glycol produced by said transesterification reaction.

5. The process for producing a symmetric dialkyl carbonate and an asymmetric dialkyl carbonate according to claim 4, wherein the process comprises the following step 1 to step 5 and said extractive distillation is performed in the step 2:
(1) a step 1 of performing a transesterification reaction of an alkylene carbonate with two or more kinds of alcohols,
(2) a step 2 of separating by distillation the reaction product in the step 1 into a low-boiling fraction mainly comprising an alcohol, a symmetric dialkyl carbonate and an asymmetric dialkyl carbonate and a high-boiling fraction mainly comprising an alkylene carbonate and an alkylene glycol,
(3) a step 3 of separating by distillation the low-boiling fraction separated by distillation in the step 2, into a low-boiling fraction mainly comprising an alcohol and a high-boiling fraction mainly comprising a symmetric dialkyl carbonate and an asymmetric dialkyl carbonate,
(4) a step 4 of separating by distillation the high-boiling fraction separated by distillation in the step 2, into a low-boiling fraction mainly comprising an alkylene glycol and containing an alkylene carbonate and a high-boiling fraction mainly comprising an alkylene carbonate, and
(5) a step 5 of recycling the low-boiling fraction separated by distillation in the step 3 and the high-boiling fraction separated by distillation in the step 4, to the step 1.

6. The process for producing a symmetric dialkyl carbonate and an asymmetric dialkyl carbonate according to claim 5, wherein the alkylene glycol in the low-boiling fraction separated by distillation in said step 4 is used as the extraction solvent.

7. The process for producing a symmetric dialkyl carbonate and an asymmetric dialkyl carbonate according to claim 5 or 6, wherein the process comprises a step 6 of obtaining an alkylene glycol by hydrolyzing the alkylene carbonate in the low-boiling fraction separated by distillation in said step 4.

8. The process for producing a symmetric dialkyl carbonate and an asymmetric dialkyl carbonate according to claim 4, wherein said alkylene carbonate is ethylene carbonate, said two or more kinds of alcohols are ethanol and methanol, said symmetric dialkyl carbonate is diethyl carbonate and dimethyl carbonate, said asymmetric dialkyl carbonate is ethyl methyl carbonate, the process comprises the following step i to step v, and said extractive distillation is performed in the step ii:
(i) a step i of performing a transesterification reaction of ethylene carbonate with a mixture of ethanol and methanol,
(ii) a step ii of separating by distillation the reaction product in the step i into a low-boiling fraction mainly comprising ethanol, methanol, diethyl carbonate, dimethyl carbonate and ethyl methyl carbonate and a high-boiling fraction mainly comprising ethylene carbonate and ethylene glycol,
(iii) a step iii of separating by distillation the low-boiling fraction separated by distillation in the step ii, into a low-boiling fraction mainly comprising ethanol and methanol and a high-boiling fraction mainly comprising diethyl carbonate, dimethyl carbonate and ethyl methyl carbonate,
(iv) a step iv of separating by distillation the high-boiling fraction separated by distillation in the step ii, into a low-boiling fraction mainly comprising ethylene glycol and containing ethylene carbonate and a high-boiling fraction mainly comprising ethylene carbonate, and
(v) a step v of recycling the low-boiling fraction separated by distillation in the step iii and the high-boiling fraction separated by distillation in the step iv, to the step i.

9. The process for producing a symmetric dialkyl carbonate and an asymmetric dialkyl carbonate according to claim 8, wherein the ethylene glycol in the low-boiling fraction separated by distillation in said step iv is used as the extraction solvent.

10. The process for producing a symmetric dialkyl carbonate and an asymmetric dialkyl carbonate according to claim 8 or 9, wherein the process comprises a step vi of obtaining ethylene glycol by hydrolyzing the ethylene carbonate in the low-boiling fraction separated by distillation in said step iv.

11. A process for producing diethyl carbonate, comprising performing a transesterification reaction of ethylene carbonate or propylene carbonate with ethanol, wherein the process comprises a step of subjecting the reaction product obtained in said transesterification reaction to extractive distillation using ethylene glycol or propylene glycol as the extraction solvent to separate by distillation a fraction containing an ether compound.

12. The process for producing diethyl carbonate according to claim 11, wherein the process comprises the following step I to step V and said extractive distillation is performed in the step II:
(I) a step I of performing a transesterification reaction of ethylene carbonate or propylene carbonate with ethanol,
(II) a step II of separating by distillation the reaction product in the step I into a low-boiling fraction mainly comprising ethanol and diethyl carbonate and a high-boiling fraction mainly comprising ethylene carbonate or propylene carbonate and ethylene glycol or propylene glycol,
(III) a step III of separating by distillation the low-boiling fraction separated by distillation in the step II, into a low-boiling fraction mainly comprising ethanol and a high-boiling fraction mainly comprising diethyl carbonate,
(IV) a step IV of separating by distillation the high-boiling fraction separated by distillation in the step II, into a low-boiling fraction mainly comprising ethylene glycol or propylene glycol and containing ethylene carbonate or propylene carbonate and a high-boiling fraction mainly comprising ethylene carbonate or propylene carbonate, and
(V) a step V of recycling the low-boiling fraction separated by distillation in the step III and the high-boiling fraction separated by distillation in the step IV, to the step I.

13. The process for producing diethyl carbonate according to claim 12, wherein the ethylene glycol or propylene glycol in the low-boiling fraction separated by distillation in said step IV is used as the extraction solvent.

14. The process for producing diethyl carbonate according to claim 12 or 13, wherein the process comprises a step VI of obtaining ethylene glycol or propylene glycol by hydrolyzing the ethylene carbonate or propylene carbonate in the low-boiling fraction separated by distillation in said step IV.
